Europäisches Patentamt

European Patent Office

Office européen des brevets

(19)

(11) Numéro de publication : **0 340 103 B1**

(12) ## FASCICULE DE BREVET EUROPEEN

(45) Date de publication du fascicule du brevet :
**25.11.92 Bulletin 92/48**

(51) Int. Cl.⁵ : **A61K 7/48, // C08G69/50**

(21) Numéro de dépôt : **89401177.4**

(22) Date de dépôt : **25.04.89**

(54) **Composition cosmétique pour le maquillage contenant des microsphères de polybêta-alanine réticulée imprégnées d'un alcool polyhydrique.**

(30) Priorité : **25.04.88 FR 8805447**

(43) Date de publication de la demande :
**02.11.89 Bulletin 89/44**

(45) Mention de la délivrance du brevet :
**25.11.92 Bulletin 92/48**

(84) Etats contractants désignés :
**AT BE CH DE ES FR GB GR IT LI NL SE**

(56) Documents cités :
**FR-A- 2 530 250**
**PARFUMS, COSMETIQUES, AROMES, no. 52, août-septembre 1983, pages 83-85; R. KERAUDY: "Les poudres polyamides dans lesformulations cosmétiques"**

(73) Titulaire : **L'OREAL**
**14, Rue Royale**
**F-75008 Paris (FR)**

(72) Inventeur : **Defossez, Béatrice**
**114, Bld de Grenelle**
**F-75015 Paris (FR)**
Inventeur : **Handjani née Vila, Rose-Marie**
**1, Place St Sulpice**
**F-75006 Paris (FR)**
Inventeur : **Lapoiriere, Claudine**
**3, rue de Lyser**
**F-94170 Le Perreux (FR)**
Inventeur : **Mahieu, Claude**
**90, avenue de Villiers**
**F-75017 Paris (FR)**
Inventeur : **Papantoniou, Christos**
**17, rue des Basserons**
**F-95160 Montmorency (FR)**
Inventeur : **Ser, Jean-Claude**
**11, rue de Fresnes**
**F-94150 Chevilly Larue (FR)**

(74) Mandataire : **Stalla-Bourdillon, Bernard et al**
**CABINET NONY & CIE 29, rue Cambacérès**
**F-75008 Paris (FR)**

## Description

La présente invention a pour objet une composition cosmétique pour le maquillage des lèvres et de la peau, tel qu'un fard à paupières, un fard à joues, un fond de teint ou un rouge à lèvres.

Plus particulièrement, la présente invention se rapporte à des produits cosmétiques pour le maquillage présentant d'excellentes propriétés émollientes permettant d'éviter la sécheresse de la peau et d'en améliorer sa souplesse.

Ces propriétés émollientes sont généralement obtenues en cosmétique par l'emploi de substances humectantes qui provoquent une réhydratation de la peau par l'eau atmosphérique que ces substances captent, celles-ci jouant donc le rôle de moyen de transfert de l'eau.

Cette action des agents humectants est connue sous l'expression anglaise de "Moisturizing".

Les substances humectantes permettant de conférer à la peau de telles propriétés émollientes sont essentiellement des alcools polyhydriques notamment le glycérol, l'éthylène glycol, le propylène glycol et le sorbitol.

Ces substances humectantes sont généralement ajoutées à des compositions cosmétiques telles que des crèmes, en particulier du type huile-dans-l'eau, dans un double but à savoir d'une part de conférer à la peau des propriétés émollientes c'est-à-dire la rendre plus douce et plus souple et d'autre part de régler les échanges d'humidité entre le produit et l'air de façon à éviter à ce que la composition n'ait tendance à sécher.

Dans la pratique, de tels agents humectants ne peuvent être employés avec efficacité que dans des compositions contenant une certaine proportion d'eau dans la mesure où ceux-ci y sont solubles.

Les essais qui ont été effectués en vue d'incorporer dans des compositions anhydres de tels agents humectants, comme le glycérol n'ont pas permis d'aboutir à des résultats satisfaisants car le glycérol n'est pas miscible aux substances grasses utilisées dans ces compositions et a donc tendance au cours du temps à migrer hors de la composition.

A la suite de divers travaux dans ce domaine, la présente invention propose une solution très satisfaisante, en vue d'obtenir des compositions de maquillage anhydres présentant une bonne action émolliente sur la peau, par l'utilisation de microsphères poreuses imprégnées d'un alcool polyhydrique, tel que le glycérol par exemple.

Les études effectuées ont en effet permis de montrer que les compositions de maquillage anhydres selon l'invention contenant ces microsphères étaient particulièrement stables au stockage et conféraient par application sur la peau d'excellentes propriétés émollientes ce qui jusqu'à présent n'avait pu être obtenu.

La présente invention a pour objet à titre de produit industriel nouveau une composition cosmétique pour le maquillage, anhydre et stable, présentant une bonne action émolliente sur la peau, cette composition contenant, en mélange avec les ingrédients usuels, une quantité suffisante de microsphères de poly β-alanine réticulée imprégnées d'un alcool polyhydrique.

Selon l'invention, l'alcool polyhydrique peut être un composé ayant de 2 à 8 atomes de carbones et de 2 à 6 fonctions hydroxy.

Parmi ces composés on peut citer : l'éthylène glycol, le glycérol, le propanediol-1,2, la diglycérine, l'érythritol, l'arabitol, l'adonitol, le sorbitol et le dulcitol.

La poly β-alanine réticulée est un polymère connu dont la préparation a été décrite dans le brevet français n°83.11609 (2.530.250).

Selon ce brevet, on prépare tout d'abord par polymérisation à partir d'acrylamide, la poly β-alanine, soluble dans l'eau puis dans un deuxième temps l'on réticule, en suspension dans un solvant organique une solution aqueuse de poly β-alanine à l'aide d'un agent réticulant tel que notamment le glutaraldéhyde.

Les microsphères de poly β-alanine réticulées obtenues sont alors soumises à une opération de tamisage en vue d'obtenir des sphères possédant une bonne homogénéité de taille.

Selon ce procédé, les microsphères sont formées lors de l'étape de réticulation et leur taille dépend essentiellement de la nature et de la quantité de l'agent de suspension utilisé.

Les microsphères de poly β-alanine réticulée peuvent également être obtenues selon un autre procédé ayant fait l'objet de la demande de brevet n°87.17573 (non publié), ce procédé permettant d'obtenir des microsphères ayant une très faible dispersité de taille ce qui permet d'éviter l'opération de tamisage.

Ce procédé consiste essentiellement:

a) à polymériser de l'acrylamide dans un mélange solvant tert-butanol/toluène, en présence d'un initiateur de polymérisation et du copolymère octadécène-anhydride maléique comme agent de suspension,

b) à soumettre la suspension de microsphères de poly β-alanine obtenues à une réticulation à l'aide d'un dialdéhyde comme réticulant tel que le glutaraldéhyde, et

c) à isoler les microsphères de poly β-alanine réticulée obtenues.

La faible dispersité de taille des microsphères de poly β-alanine réticulée est due essentiellement à la nature et à la quantité du mélange solvant utilisé lors de la polymérisation de l'acrylamide.

De préférence, le mélange solvant tert-butanol/toluène est utilisé dans des rapports compris entre 1:24 et 10:1 et de préférence entre 1:6 et 6:1.

L'initiateur de polymérisation est de préférence du tert-butylate de sodium ou de potassium utilisé en une proportion d'environ 0,1 à environ 2 moles/% par rapport à l'acrylamide.

La température de polymérisation est de l'ordre de 60°C à environ 100°C mais de préférence d'environ 80°C.

Ce dernier procédé est tout particulièrement avantageux dans la mesure où il permet d'obtenir avec une bonne reproductibilité des microsphères de poly β-alanine réticulée ayant une très faible dispersité de taille.

Les microsphères obtenues peuvent, si nécessaire, être soumises à une réaction de réduction par exemple par le borohydrure de sodium ou tout autre agent réducteur similaire, en vue de réduire les fonctions aldéhydes libres qui pourraient subsister.

Pour les compositions de maquillage selon l'invention, on utilise de préférence des microsphères de poly β-alanine réticulée ayant un diamètre moyen compris entre 0,1 et 7μm.

L'imprégnation des microsphères de poly β-alanine réticulée à l'aide d'un alcool polyhydrique peut être réalisée suivant plusieurs procédés. Par exemple les microsphères de poly β-alanine réticulée sont mises en contact avec un alcool polyhydrique dans un récipient muni d'une puissante agitation. Le mélange initialement pâteux prend progressivement la forme d'un gel puis est désagrégé jusqu'à l'obtention d'une poudre d'aspect homogène.

Il est également possible d'imprégner, par un alcool polyhydrique, des microsphères de poly β-alanine réticulée contenant de l'eau, cette eau étant ensuite éliminée par un procédé de séchage classique tel que la lyophilisation, séchage sous vide, etc... Bien entendu ces procédés sont décrits à titre indicatif et n'ont aucun caractère limitatif.

Selon l'invention, les microsphères de poly β-alanine réticulée sont généralement imprégnées entre 10 et 600% en poids de l'alcool polyhydrique.

Le pourcentage de microsphères de poly β-alanine réticulée imprégnées d'alcool polyhydrique dans les compositions cosmétiques de maquillage, est généralement compris entre 0,1 et 60% mais de préférence entre 0,5 et 40% en poids par rapport au poids total de la composition.

Il va de soi que le pourcentage choisi dépendra bien entendu de l'effet recherché et du taux d'imprégnation des microsphères.

Comme indiqué précédemment les compositions cosmétiques de maquillage selon l'invention peuvent se présenter sous forme d'un fard à paupières, d'un fard à joues, d'un fond de teint, d'un rouge à lèvres, d'une poudre libre ou encore sous forme d'une poudre compacte.

Lorsque les compositions selon l'invention sont sous forme d'un fard à paupières, d'un fard à joues ou d'un fond de teint, elles peuvent se présenter soit sous forme d'une poudre compactée ou non, soit sous forme d'un produit contenant des corps gras et éventuellement des solvants organiques.

Lorsque les compositions se présentent sous forme d'une poudre, compactée ou non, elles sont généralement constituées:
- de 0,1 à 60% de microsphères de poly β-alanine récticulée imprégnées d'alcool polyhydrique,
- de 0 à 20% d'un corps gras,
- de 1 à 70% de pigment(s) coloré(s), et
- de 5 à 90% d'une charge minérale ou organique tel que du talc, de l'amidon etc...

Lorsque les compositions se présentent sous forme d'un produit gras, elles sont généralement constituées:
- de 0,1 à 60% de microsphères de poly β-alanine réticulée imprégnées d'alcool polyhydrique,
- de 5 à 98% d'un corps gras,
- de 0 à 80% d'un solvant, et
- de 1 à 30% de pigment(s) coloré(s).

Selon cette dernière forme de réalisation, les compositions peuvent également contenir des charges minérales ou organiques telles que mentionnées ci-dessus.

Lorsque les compositions se présentent sous forme d'un rouge à lèvres, coloré ou non, elles sont généralement constituées:
- de 0,1 à 60% de microsphères de poly β-alanine réticulée imprégnées d'alcool polyhydrique,
- de 20 à 95 % d'un corps gras,
- de 0,2 à 10% d'une charge minérale ou organique, et
- de 0 à 25% de pigment(s) coloré(s) mais de préférence de 0 à 20%.

Selon ces différentes formes de réalisation, le corps gras est au moins une huile ou un mélange d'au moins une huile et d'au moins une cire.

Parmi les huiles susceptibles d'être utilisées dans les compositions selon l'invention on peut en particulier citer:

- les huiles minérales telles que l'huile de paraffine, l'huile de vaseline et les huiles minérales ayant un point d'ébullition compris entre 310 et 410°C,
- les huiles d'origine animales telles que le perhydrosqualène,
- les huiles végétales telles que l'huile d'amande douce, l'huile de calophyllum, l'huile de palme, l'huile d'avocat, l'huile de jojoba, l'huile d'olive, l'huile de ricin, les huiles de germes de céréales telles que l'huile de germes de blé,
- les huiles de silicone telles que le diméthylpolysiloxane,
- les esters de synthèse tels que l'huile de Purcelin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le di-caprylate de propylène glycol, l'adipate de di-isopropyle,
- les alcools organiques tels que l'alcool oléique, l'alcool linoléique, l'alcool linolénique, l'alcool isostéarylique, l'octyl dodécanol,
- les esters dérivés d'acide lanolique tels que le lanolate d'isopropyle, le lanolate d'isocétyle,
- parmi les huiles, on peut également citer: les acétylglycérides, les octanoates et décanoates d'alcools et de polyalcools tels que ceux de glycol et de glycérol, les ricinoléates d'alcools et de polyalcools tels que celui de cétyle,

Parmi les cires susceptibles d'être utilisées dans les compositions selon l'invention on peut mentionner:
- les cires minérales telles que les cires microcristallines, la paraffine, le petrolatum, la vaseline,
- les cires fossiles telles que l'ozokérite, la cire de montan,
- les cires d'origine animale telles que la cire d'abeille, le spermaceti, la cire de lanoline, les dérivés issus de la lanoline tels que les alcools de lanoline, la lanoline hydrogénée, la lanoline hydroxylée, la lanoline acétylée, les acides gras de la lanoline, l'alcool de lanoline acétylée,
- les cires d'origine végétale telles que la cire de candellila, la cire de Carnauba, la cire du Japon, le beurre de cacao,
- les huiles hydrogénées concrètes à 25° telles que l'huile de ricin hydrogénée, l'huile de palme hydrogénée, le suif hydrogéné, l'huile de coco hydrogénée,
- les cires synthétiques telles que les cires de polyéthylène, les cires de polyéthylène copolymérisées,
- les esters gras concrets à 25°C tels que le monomyristate de propylène glycol, le myristate de myristyle,
- les cires de silicone telles que le méthyloctadécaneoxypoly-siloxane et le poly(diméthylsiloxy) stéaroxysiloxane,
- parmi les cires on peut également citer: l'alcool cétylique, l'alcool stéarylique, les mono, di et triglycérides concrets à 25°C, le monoéthanolamide stéarique, la colophane et ses dérivés tels que les abiétates de glycol et de glycérol, les sucro glycérides et les oléates, myristates, lanolates, stéarates et dihydroxystéarates de calcium, magnésium, zinc et aluminium.

Parmi les solvants susceptibles d'être utilisés dans les compositions se présentant sous forme grasse anhydre, on peut en particulier mentionner: les isoparaffines, les silicones cycliques ou linéaires à point d'ébullition inférieur à 200°C, les solvants chlorés, etc...

Comme pigments colorés on peut mentionner le noir de carbone ou l'oxyde de fer noir, les oxydes de chrome, les oxydes de fer jaune et rouge, les outremers (polysulfures d'aluminosilicates), le pyrophosphate de manganèse, le bleu ferrique, le bioxyde de titane et enfin certaines poudres métalliques telles que celles d'argent ou d'aluminium. Les pigments sont le plus souvent utilisés en mélange avec des agents nacrants tels que l'oxychlorure de bismuth, le mica-titane, les cristaux de guanine et certains colorants organiques tels que le carmin de cochenille et les laques organiques.

Ces laques qui sont couramment employées pour conférer aux lèvres et à la peau un effet de maquillage, sont des sels de calcium, de baryum, d'aluminium ou de zirconium, de colorants acides tels que les colorants halogéno-acides, azoïques, anthraquinoniques, etc...

Parmi ces laques on peut en particulier citer celles connues sous les dénominations de D and C Red 21, D and C Orange 5, D and C Red 27, D and C Orange 10, D and C Red 3, D and C Red 7, D and C Red 2, D and C Red 4, D and C Red 8, D and C Red 33, D and C Yellow 5, D and C Yellow 6, D and C Green 5, D and C Yellow 10, D and C Green 3, D and C Blue 1, D and C Blue 2, D and C Violet 1, etc...

Les compositions cosmétiques de maquillage selon l'invention peuvent également contenir des agents antioxydants tels que les esters propylique, octylique, et dodécylique de l'acide gallique, le butylhydroxytoluène, et le butylhydroxyanisole ainsi que des parfums et des agents conservateurs tels que le parahydroxybenzoate de méthyle ou de propyle.

On va maintenant donner à titre d'illustration et sans aucun caractère limitatif un exemple de préparation des microsphères de poly β-alanine réticulée puis des exemples d'imprégnation par un alcool polyhydrique ainsi que plusieurs exemples de compositions cosmétiques de maquillage anhydres selon l'invention.

PREPARATION DES MICROSPHERES DE POLY β-ALANINE RETICULEE IMPREGNEES D'UN ALCOOL POLYHYDRIQUE INFERIEUR

A - Préparation de la poly β-alanine.

Dans un réacteur de 3 litres muni d'un agitateur de type A "Ancre" d'un diamètre de 90mm, d'une arrivée d'azote, d'une ampoule d'addition et d'une tête de colonne à distiller on introduit: 1125g de toluène, 444g de tert-butanol et 0,75g de copolymère octadécène-anhydride maléique (vendu sous la dénomination de "PA-18" par la Société GULF).

Après chauffage de ce mélange à 70°C on ajoute 150g d'acrylamide. On porte alors à la température de 100°C et on distille 90ml du mélange azéotrope eau/toluène/tert-butanol. Après la fin de la distillation, on refroidit le mélange réactionnel à 80°C, et on ajuste la vitesse d'agitation à 600t/min.

On ajoute alors en 10 min une solution de 3,36g de tert-butylate de potassium dans 62g de tert-butanol. L'ampoule d'addition est rincée par 75g de toluène. Après agitation pendant 5 heures à 80°C, on laisse revenir à la température ambiante. On ajoute ensuite goutte à goutte au mélange 11,25ml d'acide chlorhydrique concentré.

B - Préparation de la poly β-alanine réticulée.

A la suspension de microsphères de poly β-alanine ainsi obtenue, on ajoute 150ml d'eau, sous vive agitation (600t/min) à une température de 50°C, puis en 15min, 18g d'une solution aqueuse à 25% de glutaraldéhyde. Après avoir maintenu l'agitation pendant 4 heures à cette température, on laisse revenir la suspension à température ambiante. Après décantation, les solvants surnageants sont éliminées et les microsphères sont lavées deux fois par 500ml d'éthanol. L'essorage après chaque lavage est effectué par centrifugation (3500t/min). Un lavage par 15 litres d'eau est ensuite effectué en continu puis l'eau est éliminée jusqu'à un volume final de mélange de 600ml. Le polymère gonflé est enfin séché par lyophilisation et l'on obtient 132g de poudre blanche constituée de microsphères dont le diamètre moyen de $4,05 \pm 2,02$ μm est déterminé par la technique d'analyse d'images sur un appareil "QUANTIMET 900" de la Société CAMBRIDGE INSTRUMENTS CO.

C - Réduction des fonctions aldéhydes résiduelles de la poly β-alanine réticulée.

A 150g de microsphères de poly β-alanine réticulée obtenues ci-dessus, on ajoute 2,2 litres d'eau et on homogénéise par agitation. Après refroidissement à une température comprise entre 5 et 10°C, on ajoute lentement une solution refroidie de borohydrure de sodium dans l'eau (5,2g de $NaBH_4$ dans 600ml d'eau refroidie à 5°C). On maintient le milieu réactionnel entre 5 et 10°C pendant 5 heures puis on amène le pH à 7 par addition d'acide acétique.

Après centrifugation du mélange et dispersion du résidu solide dans 450ml d'eau, on le soumet à un lavage et continu par 5 litres d'eau (lavage en cellule du type "AMICON" équipée de filtre DIAPOR 0,2μm, pression 200.000 Pa, agitation durant la totalité du lavage). Les microsphères hydratées sont ensuite séchées par lyophilisation. L'absence de coloration en présence de réactif de SCHIFF permet de conclure que les fonctions aldéhydes résiduelles ont été réduites. Après analyse, le diamètre des microsphères est identique aux microsphères de départ.

D - Imprégnation des microsphères de poly β-alanine réticulée

1. On introduit dans un récipient muni d'une puissante et lente agitation 100g de microsphères de poly β-alanine réticulée telles qu'obtenues ci-dessus et 300g de glycérol. Le mélange initialement pâteux prend progressivement la forme d'un gel qui est ensuite désagrégé par agitation jusqu'à l'obtention d'une poudre d'aspect homogène, après deux heures de malaxage, à la température ambiante et à l'abri de l'humidité atmosphérique.

2. On introduit dans un gel composé de 5g de poly β-alanine réticulée préparée comme mentionné ci-dessus et de 50g d'eau, 15g de glycérol. Après homogénéisation le mélange est concentré à l'évaporateur rotatif à 50-60°C sous 2700 Pa jusqu'à l'obtention d'un poids constant.

3. On introduit dans un récipient muni d'une puissante et lente agitation 100g de microsphères de poly β-alanine réticulée telles qu'obtenues ci-dessus et 150g d'une solution à environ 70% en poids de sorbitol. Après mélange on constate la formation d'un mélange pâteux qui est ensuite désagrégé, jusqu'à l'obtention d'une poudre homogène, par agitation à la température ambiante et à l'abri de l'humidité atmosphérique.

Selon ces mêmes modes opératoires, il est possible d'obtenir des microsphères de poly β-alanine réticulée imprégnées à l'aide d'autres alcools polyhydriques et à différents taux en faisant varier la quantité de l'alcool polyhydrique à imprégner dans les limites comprises entre 10 et 600% en poids de l'alcool polyhydrique par rapport au poids de la poly β-alanine réticulée.

EXEMPLES DE COMPOSITIONS DE MAQUILLAGE

EXEMPLE 1

Fard à paupières

- Microsphères de poly $\beta$-alanine
  réticulée imprégnées de glycérol à 300%
  suivant l'exemple D-1..................... 5%
- Pigments................................. 30%
- Mica-titane.............................. 50%
- Lanoline liquide......................... 5%
- Huile de vaseline........................ 4,8%
- Parahydroxybenzoate de propyle........... 0,2%

- Talc q.s.p............................... 100%

EXEMPLE 2

Fard à joues

- Microsphères de poly $\beta$-alanine
  réticulée imprégnées de sorbitol à 100%
  suivant l'exemple D-3..................... 4%
- Carbonate de magnésie.................... 1%
- Amidon................................... 10%
- Stéarate de zinc......................... 2%
- Mica-titane.............................. 2%
- Lanoline liquide......................... 2%
- Huile de vaseline........................ 2,9%
- Parahydroxybenzoate de propyle........... 0,1%
- Pigments ................................ 30%
- Parfum................................... 1%

- Talc q.s.p............................... 100%

EP 0 340 103 B1

EXEMPLE 3

<u>Rouge à lèvres</u>

- Microsphères de poly $\beta$-alanine réticulée
imprégnées de glycérol à 300%
selon l'exemple D-1........................ 20g
- Ozokérite................................ 12g
- Cire microcristalline.................... 4g
- Cire de Candellila....................... 6g
- Huile de jojoba.......................... 5g
- Huile de ricin........................... 1g
- Lanoline liquide......................... 15g
- Lanoline acétylée........................ 8g
- Huile de vaseline........................ 9g
- Talc..................................... 3g
- Mica-titane.............................. 7g
- D and C Red 7 Ca lake.................... 4,2g
- D and C Red 6 Ba lake.................... 2,3g
- F D C Yellow 5........................... 0,8g


- Bioxyde de titane........................ 2,5g
- Butylhydroxytoluène...................... 0,2g
- Parfum q.s

EXEMPLE 4

<u>Fard à paupières</u>

- Microsphères de poly $\beta$-alanine réticulée
imprégnées de sorbitol à 100%
suivant l'exemple D-3..................... 3g
- Cire de Candellila....................... 9g
- Cire microcristalline.................... 8g
- Beurre de cacao.......................... 13g
- Myristate d'isopropyle................... 34,9g
- Talc..................................... 7g
- Mica-titane.............................. 10g
- Bioxyde de titane........................ 10g
- Oxydes de fer............................ 5g
- Butylhydroxytoluène...................... 0,1g

7

EXEMPLE 5

<u>Fard à joues</u>

- Microsphères de poly β-alanine réticulée
  imprégnées de glycérol à 300%
  suivant l'exemple D-2....................... 2g
- Huile de paraffine........................ 50,65g
- Petrolatum................................ 10g
- Cire de Carnauba.......................... 5g
- Ozokérite................................. 5g
- Mica-titane............................... 10g
- Bioxyde de titane......................... 5g
- D and C Red 7............................. 0,2g
- Oxydes de fer............................. 2g
- Parahydroxybenzoate de propyle............ 0,1g
- Butylhydroxytoluène....................... 0,05g

**Revendications**

1. Composition cosmétique pour le maquillage, anhydre et stable, présentant une bonne action émolliente sur la peau, caractérisée par le fait qu'elle contient, en mélange avec las ingrédients usuels, une quantité suffisante de microsphères de poly β-alanine réticulée imprégnées d'un alcool polyhydrique ayant de 2 à 8 atomes de carbone et de 2 à 6 fonctions hydroxy.

2. Composition selon la revendication 1, caractérisée par le fait que les microsphères ont un diamètre compris entre 0,1 et 7μm et sont imprégnées entre 10 et 600% en poids de l'alcool polyhydrique.

3. Composition selon la revendication 1 ou 2 caractérisée par le fait que l'alcool polyhydrique est l'éthylène glycol, le glycérol, le propanediol-1,2, la diglycérine, l'érytritol, l'arabitol, l'adonitol, le sorbitol ou le dulcitol.

4. Composition selon l'une quelconque des revendications précédentes, caractérisée par le fait qu'elle contient entre 0,1 et 60% en poids mais de préférence entre 0,5 et 40% en poids de microsphères par rapport au poids total de la composition.

5. Composition selon l'une quelconque des revendications précédentes, caractérisée par le fait qu'elle se présente sous forme d'une poudre compactée ou non, constituée:
   - de 0,1 à 60% de microsphères de poly β-alanine réticulée imprégnées d'alcool polyhydrique,
   - de 0 à 20% d'un corps gras,
   - de 1 à 70% de pigment(s) coloré(s), et
   - de 5 à 90% d'une charge minérale ou organique.

6. Composition selon l'une quelconque des revendications 1 à 4, caractérisée par le fait qu'elle se présente sous forme d'un produit gras constitué:
   - de 0,1 à 60% de microsphères de poly β-alanine réticulée imprégnées d'alcool polyhydrique,
   - de 5 à 98% d'un corps gras,
   - de 0 à 80% d'un solvant, et
   - de 1 à 30% de pigment(s) coloré(s).

7. Composition selon l'une quelconque des revendications 1 à 4, caractérisée par le fait qu'elle se présente sous forme d'un rouge à lèvres, coloré on non, constitué:

- de 0,1 à 60% de microsphères de poly β-alanine réticulée imprégnées d'un alcool polyhydrique,
- de 20 à 95% d'un corps gras,
- de 0,2 à 10% d'une charge minérale ou organique, et
- de 0 à 25% de pigment(s) coloré(s) mais de préférence de 0 à 20%.

**8.** Composition selon l'une quelconque des revendications précédentes, caractérisée par le fait qu'elle contient en outre au moins un agent antioxydant, un parfum ou un agent conservateur.

## Claims

**1.** Cosmetic composition for make-up, anhydrous and stable, exhibiting a good emollient action on the skin, characterised in that it contains, mixed with the usual ingredients, a sufficient quantity of microspheres of crosslinked poly-β-alanine which are impregnated with a polyhydric alcohol containing from 2 to 8 carbon atoms and from 2 to 6 hydroxyl functional groups.

**2.** Composition according to Claim 1, characterised in that the microspheres have a diameter of between 0.1 and 7 μm and are impregnated between 10 and 600 % by weight with the polyhydric alcohol.

**3.** Composition according to Claim 1 or 2, characterised in that the polyhydric alcohol is ethylene glycol, glycerol, 1,2-propanediol, diglycerine, erythritol, arabitol, adonitol, sorbitol or dulcitol.

**4.** Composition according to any one of the preceding claims, characterised in that it contains between 0.1 and 60 % by weight but preferably between 0.5 and 40 % by weight of microspheres relative to the total weight of the composition.

**5.** Composition according to any one of the preceding claims, characterised in that it is in the form of a compacted or uncompacted powder consisting:
- of 0.1 to 60 % of microspheres of crosslinked poly-β-alanine which are impregnated with polyhydric alcohol,
- of 0 to 20 % of a fatty substance,
- of 1 to 70 % of coloured pigment(s), and
- of 5 to 90 % of an inorganic or organic filler.

**6.** Composition according to any one of Claims 1 to 4, characterised in that it is in the form of a fatty product consisting;
- of 0.1 to 60 % of microspheres of crosslinked poly-β-alanine which are impregnated with polyhydric alcohol,
- of 5 to 98 % of a fatty substance,
- of 0 to 80 % of a solvent, and
- of 1 to 30 % of coloured pigment(s).

**7.** Composition according to any one of Claims 1 to 4, characterised in that it is in the form of a lipstick, coloured or otherwise, consisting:
- of 0.1 to 60 % of microspheres of crosslinked poly-β-alanine which are impregnated with a polyhydric alcohol,
- of 20 to 95 % of a fatty substance,
- of 0.2 to 10 % of an inorganic or organic filler, and
- of 0 to 25 % of coloured pigment(s), but preferably of 0 to 20 %.

**8.** Composition according to any one of the preceding claims, characterised in that it additionally contains at least an antioxidant agent, a perfume or a preserving agent.

## Patentansprüche

**1.** Wasserfreie, stabile kosmetische Zubereitung zum Schminken, die der Haut Geschmeidigkeit verleiht, dadurch gekennzeichnet, daß sie in Mischung mit üblichen Bestandteilen eine ausreichende Menge von vernetzten Poly-β-alanin-Mikrokügelchen enthält, die mit einem mehrwertigen Alkohol imprägniert sind, welcher 2 bis 8 Kohlenstoffatome und 2 bis 6 Hydroxyfunktionen aufweist.

2. Zubereitung nach Anspruch 1, dadurch gekennzeichnet, daß die Mikrokügelchen einen Durchmesser zwischen 0, 1 und 7 µm besitzen und mit 10 bis 600 Gew.-% des mehrwertigen Alkohols imprägniert sind.

3. Zubereitung nach Anspruch 1 oder 2, dadurch gekennzeichnet, daß der mehrwertige Alkohol Ethylenglykol, Glycerin, 1,2-Propandiol, Diglycerin, Erythrit, Arabit, Adonit, Sorbit oder Dulcit ist.

4. Zubereitung nach einem der vorangehenden Ansprüche, dadurch gekennzeichnet, daß sie zwischen 0, 1 und 60 Gew.-%, vorzugsweise zwischen 0,5 und 40 Gew.-%, Mikrokügelchen enthält, bezogen auf das Gesamtgewicht der Zubereitung.

5. Zubereitung nach irgendeinem der vorangehenden Ansprüche, dadurch gekennzeichnet, daß sie in Form eines verfestigten oder nicht verfestigten Pulvers vorliegt und folgende Komponenten enthält:
   - 0, 1 bis 60 % vernetzte Poly-β-alanin-Mikrokügelchen, die mit einem mehrwertigen Alkohol imprägniert sind;
   - 0 bis 20 % Fettsubstanz;
   - 1 bis 70 % Farbpigment(e); und
   - 5 bis 90 % mineralischer oder organischer Füllstoff.

6. Zubereitung nach irgendeinem der Ansprüche 1 bis 4, dadurch gekennzeichnet, daß sie in Form eines Fettproduktes vorliegt und folgende Komponenten enthält:
   - 0,1 bis 60 % vernetzte Poly-β-alanin-Mikrokügelchen, die mit einem mehrwertigen Alkohol imprägniert sind;
   - 5 bis 98 % Fettsubstanz;
   - 0 bis 80 % Lösungsmittel; und
   - 1 bis 30 % Farbpigment(e).

7. Zubereitung nach irgendeinem der Ansprüche 1 bis 4, dadurch gekennzeichnet, daß sie in Form eines gefärbten oder nicht gefärbten Lippenstiftes vorliegt und folgende Komponenten enthält:
   - 0,1 bis 60 % vernetzte Poly-β-alanin-Mikrokügelchen, die mit einem mehrwertigen Alkohol imprägniert sind;
   - 20 bis 95 % Fettsubstanz;
   - 0,2 bis 10 eines mineralischen oder organischen Füllstoffes; und
   - 0 bis 25 %, bevorzugt 0 bis 20 %, Farbpigment(e).

8. Zubereitung nach einem der vorangehenden Ansprüche, dadurch gekennzeichnet, daß sie außerdem mindestens einen Oxidationsinhibitor, einen Duftstoff oder ein Konservierungsmittel enthält.